# FASCICULE DE BREVET EUROPEEN

(11) **EP 0 491 921 B1**
(45) Date de publication et mention de la délivrance du brevet: **07.02.1996**
(21) Numéro de dépôt: 91913037.7
(22) Date de dépôt: 12.07.1991
(51) Int. Cl.: A61K 47/42, A61K 7/48

(54) **PROCEDE DE PREPARATION D'UNE LIPOPROTEINE MODIFIEE PAR INCORPORATION D'UNE SUBSTANCE ACTIVE LIPOPHILE**
VERFAHREN ZUR HERSTELLUNG EINES DURCH EINBAU EINER LIPOPHILEN WIRKSUBSTANZ MODIFIZIERTEN LIPOPROTEINS
METHOD FOR PREPARING A LIPOPROTEIN MODIFIED BY THE INCORPORATION OF A LIPOPHILIC ACTIVE SUBSTANCE

(30) Priorité: 13.07.1990 FR 9008980
(43) Date de publication de la demande: 01.07.1992
(73) Titulaire: UNIVERSITE DROIT ET SANTE LILLE II, F-59800 Lille (FR); UNIVERSITE PAUL SABATIER (TOULOUSE III), F-31062 Toulouse Cédex (FR)
(72) Inventeur: FAVRE, Gilles, F-31300 Toulouse (FR); DURIEZ, Patrick, F-59800 Lille (FR); MONARD, Françoise, F-59800 Lille (FR); MEDHI, Samadi-Baboli, F-31810 Vénerque (FR); SOULA, Georges, F-31400 Toulouse (FR); FRUCHART, Jean-Charles, F-59320 Ennetières-en-Weppes (FR)
(74) Mandataire: Tonnellier, Jean-Claude
(86) Numéro de dépôt international: FR9100573
(87) Numéro de publication internationale: WO9200761

(56) Documents cités:
- EP-A- 0 277 849
- WO-A-86/07540
- WO-A-87/01035
- WO-A-88/09345
- FR-A- 2 631 236
- Chemical Abstracts, vol. 102, no. 15, 15 avril 1985, Columbus, Ohio, US; E. Granot et al.: "Core modification of human low-density lipoprotein by artificial triacylglycerol emulsion", voir page 245, résumé 127579a
- Chemical Abstracts, vol. 113, no. 22, 26 novembre 1990, Columbus, Ohio, US; M. Samadi-Baboli et al.: "Comparative study of the incorporation of ellipticine esters into low density lipoprotein (LDL) and selective cell uptake of drug-LDL complex via the LDL receptor pathway in vitro", voir page 410, résumé 197780k
- Journal of Medicinal Chemistry, vol. 25, no. 10, octobre 1982, American Chemical Society; R.E. Counsell et al.: "Lipoproteins as potential site-specific delivery systems for diagnostic and therapeutic agents", pages 1115-1120, voir page 1118, colonne 2, paragraphe 2

## Description

La présente invention a pour objet un procédé de préparation d'une lipoprotéine modifiée par incorporation d'une substance active lipophile.

On sait qu'actuellement l'un des objectifs de la recherche pharmaceutique est de diriger les médicaments vers des organes et/ou des cellules cibles, afin d'augmenter l'efficacité thérapeutique tout en réduisant les effets secondaires. L'un des moyens pour atteindre cet objectif consiste à lier le médicament à une molécule, par exemple une macromolécule, capable de jouer le rôle de transporteur (ou "vecteur") du médicament et de conduire celui-ci spécifiquement dans l'organe-cible ou les cellules-cibles.

On a déjà utilisé ou proposé, comme transporteur de substance biologiquement active, diverses macromolécules ou associations de molécules telles que des liposomes, des nanocapsules, de l'ADN, des lectines, des anticorps et des lipoprotéines.

On sait que les lipoprotéines constituent un groupe important de protéines sériques comprenant un noyau (ou "core") lipidique entouré d'une enveloppe contenant notamment des phospholipides et des protéines spécifiques (apo-lipoprotéines).

Les lipoprotéines constituent des systèmes de stockage et de transport des lipides, et en particulier du cholestérol.

On sait que les lipoprotéines sont classées en fonction de leur densité. On distingue :
- les chylomicrons qui sont des particules de densité inférieur à 0,96 ;
- les lipoprotéines de très faible densité (VLDL), de densité comprise entre 0,96 et 1,006 g/cm³ ;
- les lipoprotéines de faible densité (LDL), de densité comprise entre 1,019 et 1,063 environ ;
- les lipoprotéines de haute densité (HDL), de densité comprise entre 1,063 et 1,21 environ.

Les lipoprotéines ont un tropisme marqué pour diverses cellules qui possèdent des récepteurs pour les apo-lipoprotéines ; en outre, dans certains cas, elles peuvent être capturées par les cellules du système réticulo-endothélial (cellules phagocytaires).

On conçoit donc que les lipoprotéines sont susceptibles de constituer des transporteurs de médicament intéressants. On a déjà proposé l'utilisation des lipoprotéines, et en particulier des LDL, comme vecteurs de médicament ; voir par exemple la demande de brevet internationale PCT n° WO 86/07540 et la demande de brevet européen n° 0277849.

Les lipoprotéines de basse densité (LDL), qui ont été le plus étudiés comme vecteurs de médicaments, sont des particules sphériques dont le diamètre est de l'ordre de 22nm. Chaque particule comprend un "core" composé d'environ 1500 molécules d'esters de cholestérol, et une enveloppe constituée de cholestérol, de phospholipides et d'apo-lipoprotéines (apo-lipoprotéine B ou apo B).

Les cellules de mammifères ont des récepteurs qui reconnaissent l'apo B et lient les LDL à leur surface. Après leur liaison à la surface membranaire des cellules, les LDL sont internalisées par endocytose et transportées aux lysosomes pour y être dégradées et répondre ainsi aux besoins de la cellule, notamment en cholestérol.

Si on utilise des LDL natives, c'est-à-dire non modifiées chimiquement, le complexe LDL-médicament se comporte comme les LDL natives, c'est-à-dire qu'il restera dans la circulation sanguine pendant 2 ou 3 jours, et sera reconnu par les cellules exprimant le récepteur pour l'apo B (Brown S.M. et Goldstein J.L., Science, 232, 34-47, 1986). C'est en particulier le cas des cellules cancéreuses qui expriment un haut niveau de récepteur pour l'apo B.

On sait par ailleurs que les lipoprotéines modifiées chimiquement (acétylées, acétoacétylées, oxydées, lactosylées, etc.), sont reconnues et capturées par les cellules du système réticulo-endothélial, notamment par les macrophages. Ici encore, le complexe lipoprotéine (modifiée chimiquement)-médicament se comporte dans l'organisme comme la lipoprotéine modifiée de départ et est capturé par les macrophages par l'intermédiaire du récepteur aux LDL acétylées (Scavenger receptor) ; voir par exemple J.M. Shaw et al., Proc. Natl. Acad. Sci. USA Vol. 85, pp.6112-6116 (1988) et M.K. Bijsterbock et al., Molecular Pharmacology, 36, pp.484-486 (1989).

Dans l'organisme, le transfert des triglycérides, des esters de cholestérol et des phospholipides entre les différentes classes de lipoprotéines (LDL, VLDL, HDL, chylomicrons) est assuré par des protéines dites de transfert, qui ont une densité supérieure à celle des lipoprotéines, et qui sont donc contenues dans le culot d'ultracentrifugation du plasma humain ou animal après séparation des lipoprotéines. Un tel culot de centrifugation constitue la fraction plasmatique dite LPDS (Lipoprotein Deficient Serum), qui possède notamment les propriétés des protéines de transfert qu'elle contient. Certaines de ces protéines de transfert ont déjà été isolées et décrites ; voir par exemple A. S. Jarnagin et al., Proc. Natl. Acad. Sci. USA Vol. 84, pp.1854-1857 (1987) ; Kato et al., J. Biol. chem. (US), 264, n° 7, 4082-4087 (1989) ; Bastiras et Calvert, J. Chromatogr. Biomed. Appl. 383, n° 1, pp.27-34 (1986).

Les protéines de transfert sont capables d'assurer par exemple le transport des triglycérides depuis les VLDL vers les LDL. Elles sont également capables d'assurer le transport des triglycérides, depuis des émulsions artificielle de triglycérides vers les LDL ; voir E. Granot et al, Biochimica et Biophysica Acta 833, pp 308-315 (1985).

On a maintenant découvert que les protéines contenues dans le LPDS sont également capables d'assurer le transport de substances biologiquement actives lipophiles in vitro, autres que des triglycérides et des esters de cholestérol notamment des médicaments non physiologiques, depuis des émulsions lipidiques contenant de telles substances lipophiles, vers les lipoprotéines, avec incorporation desdites substances dans les lipoprotéines.

Ce procédé d'incorporation de substances biologiquement actives dans les lipoprotéines est intéressant car il permet d'éviter l'utilisation de substances indésirables, telles que les détergents, et grâce à l'utilisation des agents naturels de transfert, il permet d'éviter toute altération de l'activité biologique des LDL.

La présente invention a donc pour objet un procédé de préparation d'une lipoprotéine modifiée par incorporation d'au moins une substance active lipophile autre qu'un triglycéride et qu'un ester de cholestérol, caractérisé par le fait que l'on incorpore ladite substance active dans une émulsion d'une phase lipidique dans une phase continue aqueuse, que l'on ajoute à l'émulsion, en agitant, une lipoprotéine de départ et au moins une protéine de transfert de lipides, qu'on laisse incuber le mélange, et que l'on isole selon les méthodes connues la lipoprotéine dans laquelle la substance active est incorporée.

La substance active lipophile que l'on désire incorporer dans les lipoprotéines est différente des constituants de la phase lipidique de l'émulsion. Toutefois la définition donnée ci-dessus du procédé de l'invention ne signifie pas que des substances lipophiles telles que des triglycérides ou des esters de cholestérol, éventuellement présents dans la phase lipidique, ne seront pas incorporés dans les lipoprotéines en même temps que ladite substance active. En fait, une certaine proportion de ces substances lipophiles présentes dans la phase lipidique est généralement incorporée dans les lipoprotéines en même temps que la substance active.

Le procédé de l'invention peut encore présenter les caractéristiques qui vont être décrites ci-après, prises isolement ou en combinaison.

La substance active lipophile qui peut être incorporée dans les lipoprotéines grâce au procédé de l'invention peut être choisie notamment parmi les médicaments antinéoplasiques, immunostimulants, immunodépresseurs, antiviraux, antibactériens, antifongiques, antiparasitaires, etc, ou encore des substances actives cosmétiques liposolubles. Si le médicament n'est pas suffisamment lipophile pour être incorporé spontanément dans la phase lipidique de l'émulsion, il peut être modifié chimiquement pour être rendu lipophile (ou davantage lipophile), par greffage, selon les méthodes connues, d'un groupement lipophile tel que par exemple le reste acyle d'un acide gras ayant au moins 8 atomes de carbone, ou d'un groupement stéroïde, dérivé par exemple du cholane ou du cholestane et possédant une fonction réactive permettant son greffage sur la substance active à incorporer.

La phase lipidique de l'émulsion peut être toute émulsion lipidique appropriée. Par exemple, la phase lipidique comprend ou est constituée par au moins un triglycéride d'acide gras, et en particulier une huile comestible. On peut utiliser notamment les émulsions lipidiques injectables disponibles dans le commerce et utilisées pour l'alimentation parentérale en perfusion, comme l'Endolipide (marque déposée) commercialisée par les laboratoires Bruneau (France) et l'Intralipide (marque déposée) commercialisée par KabiVitrum.

On peut également utiliser une phase lipidique comprenant ou constituée par au moins un ester de cholestérol, par exemple un ester de cholestérol dérivé d'un acide carbonylique ayant 1 à 24 atomes de carbone.

On peut aussi utiliser comme phase lipidique des vésicules lipidiques telles que des liposomes ou analogues. La composition de telles vésicules lipidiques est connue et ne sera pas rappelée ici.

Parmi les médicaments pouvant être incorporés dans les lipoprotéines selon le procédé de l'invention, on citera par exemple l'elliptinium, la mitoxantrone, l'amétantrone, le méthotrexate, la doxorubicine, la daunorubicine, la vincristine, etc... ainsi que leurs dérivés d'acylation avec un acide gras ; le ketoconazole (ou un analogue dont le groupement acétyle est remplacé par un acyle d'acide gras) ; les dérivés acylés (d'acide gras) du muramyldipeptide ; les dérivés photoactivables de la porphyrine (Photofrin II).

Parmi les substances actives cosmétiques pouvant être incorporées dans les lipoprotéines, on citera par exemple les vitamines liposolubles (notamment vitamines A et E).

Parmi les acides gras dont le greffage sur la substance active facilite son incorporation dans les lipoprotéines, on citera notamment les acides gras ayant 4 à 24, et en particulier 8 à 24 atomes de carbone, saturés ou insaturés, tels que par exemple parmi les acides palmitique, stéarique, oléique, linolénique, lignocérique, etc....

L'émulsion lipidique peut être stabilisée à l'aide d'un agent émulsionnant, en particulier un phospholipide tel que la lécithine, qui n'a aucun effet de dégradation sur les protéines.

La lipoprotéine de départ peut être une lipoprotéine native, et en particulier une LDL.

Les lipoprotéines natives peuvent être séparées du plasma selon les méthodes connues et, en particulier par ultracentrifugation. Elles peuvent également être isolées du plasma par filtration sur membranes à fibres creuses. Les LDL et les VLDL peuvent également être isolées par adsorption sélective, par exemple sur des billes de cellulose-sulfate de dextran : voir S.Yokoyama, in : "Plasmapheresis : New trends in therapeutic applications, N.Y. Malchesky et JW. Smith Eds, Cleveland, ISAO Press, 231-237 (1983) ; et S. Yokoyama et al., Arteriosclerosis Vol. 5 n° 6 pp 613-622 (1985).

On peut également utiliser comme lipoprotéine de départ une lipoprotéine modifiée chimiquement pour permettre sa reconnaissance par le système réticulo-endothélial. On utilise à cet effet des lipoprotéines modifiées selon les méthodes connues par exemple, des LDL acétylés, acéto-acétylés ou oxydées ; voir notamment JM. Shaw, article cité ; Basu et al., Proc. Natl. Acad. Sci USA 73, 3178-3182 (1976) ; Steinbrechert, J. Biol. Chem. 262, 3603 (1987) ;

La protéine de transfert utilisée dans le procédé de l'invention peut être soit une protéine humaine de transfert de lipide connue (voir la littérature citée ci-dessus), soit plus généralement une protéine de transfert de lipide animale ou végétale. Ces dernières protéines ont été décrites notamment par BASU et al., Biochimica et Biophysica Acta, 959, n° 2, 134-142 (1988) et GROSBOIS et al., Plant Physiology, 90, n° 4, 1560-1564 (1989).

La protéine de transfert peut également être ajoutée sous la forme d'une fraction plasmatique LPDS, définie comme indiqué ci-dessus.

Pour mettre en oeuvre le procédé de l'invention, on peut préparer l'émulsion lipidique selon les méthodes usuelles, ou bien utiliser une émulsion lipidique toute préparée du commerce, habituellement utilisée pour l'alimentation parentérale par perfusion.

On peut ajouter la substance active lipophile à l'émulsion , ou inversement. Il est préférable d'agiter fortement afin d'assurer une bonne solubilisation de la substance active dans la phase lipidique.

On peut ajouter ensuite la protéine de transfert (ou la fraction LPDS), qui est soluble dans l'eau, en agitant pour assurer une homogénéité convenable.

On laisse ensuite incuber le mélange pendant un temps suffisant pour obtenir une incorporation de la substance active lipophile dans la lipoprotéine.

Les quantités de la substance active lipophile et de lipoprotéine étant données, on détermine facilement de façon expérimentale la quantité de protéine de transfert ou de LPDS nécessaire pour obtenir une incorporation suffisante de la substance active lipophile dans la lipoprotéine.

De même, la durée d'incubation nécessaire pour obtenir une incorporation suffisante ou maximale de la substance active lipophile peut être déterminée dans chaque cas par de simples expériences de routine.

Par exemple, on laisse incuber le mélange pendant 15 - 20 heures à une température de 37°C.

La lipoprotéine modifiée par incorporation de la substance active lipophile peut alors être séparée selon les techniques usuelles, par exemple par ultracentrifugation en gradient de densité ou par chromatographie d'affinité.

Ceci permet d'assurer que toute impureté ou réactif résiduel soit éliminé pour aboutir à un produit sensiblement exempt de produits de départ.

Les compositions pharmaceutiques de l'invention sont bien entendu utilisées, en médecine humaine ou vétérinaire, dans le domaine thérapeutique correspondant à celui du médicament incorporé dans les lipoprotéines. La posologie (calculée en substance active) est au plus égale, et le plus souvent inférieure, à celle du médicament qui est incorporé.

Ces compositions pharmaceutiques sont administrées par voie parentérale.

Les lipoprotéines modifiées de l'invention peuvent également contenir des substances actives lipophiles utilisables dans des compositions cosmétiques, comme des vitamines liposolubles ou d'autres principes actifs lipophiles connus pour améliorer l'aspect esthétique de la peau. Ces compositions cosmétiques peuvent se présenter notamment sous la forme de suspension aqueuse ou d'émulsion dans lesquelles les lipoprotéines modifiées de l'invention sont incorporées.

Les exemples suivants illustrent l'invention sans toutefois la limiter.

### EXEMPLE 1 : Incorporation d'un médicament lipophile à chaîne grasse dans les LDL

### a) Matériel :

Pour isoler les LDL, on part de plasma sanguin dont on a vérifié la sérologie négative vis-à-vis des virus de l'hépatite et du HIV.

Les LDL sont isolées par ultracentrifugation zonale du plasma, entre les densités 1,020-1,063. Elles sont ensuites dialysées pendant 24 heures contre une solution aqueuse d'EDTA 0,3mM, pH = 8, puis filtrées stérilement (0,22 µm) et conservées sous atmosphère d'azote.

L'émulsion d'Endolipide "20%" (Laboratoires Bruneau) a la composition suivante :

| | |
|---|---|
| - huile de soja | 20 g |
| - lécithine | 1,2 g |
| - glycérol | 2,5 g |
| - eau QSP | 100 ml. |

L'émulsion d'Endolipide est lavée volume à volume avec du sérum physiologique (solution aqueuse de NaCl à 8 g/l) puis soumise à une ultracentrifugation pendant 30 min. à 30 000 t/min., à 10°C. La fraction lipidique est alors diluée volume à volume dans du sérum physiologique.

Le but de ce lavage de l'émulsion d'Endolipide est d'éliminer une partie des phospholipides (lécithine) qui sont en fait présents en quantité supérieure à la quantité suffisante ; voir E. GRANOT et al, article cité.

La fraction LPDS, contenant les protéines de transfert, de densité supérieure à 1,21, est obtenue par ultracentrifugation pendant 24 heures à 40 000 t/min., à 4°C, d'un plasma amené à une densité de 1,21 g/cm² par addition de KBr (densité mesurée à 20°C). Cette fraction LPDS est alors dialysée trois fois pendant 12 heures contre du tampon Tris 10mM, NaCl 140 mM, EDTA 1mM, pH8, et la concentration en protéines est ajustée à 60g /litre.

Le médicament incorporé dans les LDL est un ester d'acide gras d'elliptinium (stéarate, palmitate ou oléate), un ester d'acide gras d'amétantrone (monostéarate, distéarate, monopalmitate, dipalmitate, monooléate, dioléate, monolinolénate, dilinolénate), ou un ester d'acide gras de mitoxantrone (dilinolénate). Ces esters ont été préparés selon la méthode décrite par Samadi-Baboli, Thèse, Université de Toulouse III, 1989.

### b) Méthode :

On dissout 1 mg du médicament dans la quantité minimum d'un solvant organique (chloroforme) puis on évapore le solvant sous pression réduite.

On ajoute ensuite 200 µl d'Endolipide lavé. On agite fortement le tube, avec formation d'un vortex pendant une minute, afin de dissoudre le médicament dans la phase lipidique. On ajoute ensuite les LDL, en quantité contenant 1 mg de protéines, dosées par la méthode de Lowry modifiée par Peterson ; référence LOWRY et al., J. Biol. Chem. 193, 265-275 (1951) et 5 ml de LPDS. Après agitation, le mélange est incubé pendant 18 heures à 37°C.

On sépare alors les LDL par ultracentrifugation selon un gradient de densité en KBr (centrifugation pendant 24 heures à 4°C à 40000 t/min.).

On peut également isoler les LDL modifiées par chromatographies d'affinité, selon les méthodes connues.

Les LDL modifiées sont alors dialysées contre une solution NaCl 140 mM pH8, puis filtrées stérilement (0,22 µm) et conservées sous azote.

On peut obtenir des LDL modifiées analogues en remplaçant l'Endolipide par une microémulsion obtenue de la façon suivante : on dissout 2 mg du médicament, 1 mg de stéarate de cholestérol et 2 mg de phospholipides dans 1 ml de chloroforme. On agite pour bien homogénéiser puis on évapore le solvant. On reprend le résidu par 200 microlitres d'isopropanol et on injecte la solution obtenue dans 3 ml de tampon phosphate 0,2 M, NaCl 0,15 M, pH 7,4 sous agitation vigoureuse, avec formation d'un vortex pendant au moins une minute. On termine par une gelfiltration (G 26) dans le tampon phosphate.

### c) Analyses :

Sur les LDL modifiées et natives, on a effectué les dosages suivants :
- protéines : selon la technique de Lowry modifiée par Peterson,
- lipides (cholestérol libre, cholestérol estérifié, triglycérides, phospholipides) : dosés selon les méthodes enzymatiques ; voir FRUCHART J.C. et al., Pharmacol. Biol., 24, 227-229, 1980 ; ZIEGENHORN J. et al., Clin. Chem., 26, 973-979 (1980) ; TAKAYAMA M. et al., Clinica Chim. Acta, 79, 93-98 (1977) ;
- médicaments (sur les LDL modifiées) : les esters d'amétantrone sont dosés après précipitation de l'apolipoprotéine B avec de l'isopropanol. Pour cela, 100 µl de LDL sont ajoutés à 100 µl d'isopropanol et agités pendant 1 minute puis laissés au repos pendant 1 nuit à 10°C sous agitation. Le mélange est alors centrifugé pendant 30 min. à 300 t/min.. Le surnageant est prélevé et évaporé. Le résidu est repris par 1 ml de chloroforme. On lit la densité optique à une longueur d'onde de 614 nm, et on compare à une gamme étalon du médicament.

Le dosage des esters d'elliptinium est réalisé en HPLC selon la technique décrite par Samadi-Baboli, Thèse déjà citée.

### d) Etudes pharmacologiques :

Le métabolisme des LDL modifiées a été étudié chez le lapin en comparant la cinétique de disparition plasmatique de LDL natives et celle des LDL modifiées injectées dans la circulation générale.

Les effets cytotoxiques des LDL modifiées par incorporation d'oléate d'elliptinium ont été étudiés sur des cellules L 1210 en présence de concentrations croissantes du médicament. La croissance cellulaire est mesurée après 48 heures d'incubation par comptage à l'aide d'un compteur Coulter.

Les résultats obtenus sont résumés ci-après.

### e) Protéines :

L'apolipoprotéine B des lipoprotéines modifiées conserve des propriétés antigéniques vis-à-vis des anticorps anti-LDL.

### Lipides :

Les compositions en lipides sont données en fonction de la concentration en protéines (apo B). Le contenu en apo B des LDL natives est d'une molécule d'apo B par particule de LDL. Ce contenu est inchangé après modification selon le procédé de l'invention.

Le contenu en cholestérol total des LDL modifiées selon la méthode indiquée ci-dessus est de 13%, au lieu de 39% pour les LDL natives de départ.

Le contenu en triglycérides est augmenté (41% dans les LDL modifiées, contre 8% dans les LDL natives).

Quant à la teneur en phospholipides, elle est pratiquement inchangée (18% pour les LDL modifiées contre 22% pour les LDL natives de départ).

### Mobilité électrophorétique :

La mobilité des LDL modifiées est identique à celle des LDL initiales.

### Reconnaissance du récepteur des apoprotéines B et E :

On a vérifié par des méthodes de compétition que les LDL modifiées par incorporation de dilinolénate de mitoxantrone se fixent sur les récepteurs aux apo B et E de cellules Hela, tout comme les LDL natives.

De même, avec les LDL modifiées par incorporation d'oléate d'elliptinium, il n'y a pas de différence significative pour la fixation, l'internalisation et la dégradation, entre les LDL natives et les LDL modifiées (essais sur fibroblastes de peau humaine).

### Quantités de médicament incorporée dans les LDL :

On a obtenu les incorporations suivantes :

### Elliptinium

- stéarate : 37 µm par mg de protéine
- palmitate : 58 µg par mg de protéine
- oléate : 83 µg par mg de protéine

### Amétantrone :

- non estérifiée : 1 molécule par particule de LDL
- monostéarate : 1 molécule/particule
- distéarate : 1 molécule/particule
- monopalmitate : 2 molécules/particule
- dipalmitate : 2 molécules/particule
- monooléate : 2 molécules par particule
- dioléate : 2 molécules par particule
- monolinolénate : 80 molécules par particule
- dilinolénate : 90 molécules par particule

### Mitoxantrone :

- non estérifiée : 1 molécule par particule
- dilinolénate : 22 molécules par particule

### Métabolisme :

Cette étude a montré que la décroissance plasmatique de la concentration en LDL modifiées (LDL-dilinolénate de mitoxantrone) marquées à l'iode 131 est proche de la courbe de décroissance plasmatique de LDL natives marquées par l'iode 125. Cette étude montre que les LDL modifiées par incorporation du médicament ne sont pas captées par le système réticulo-endothélial.

### Cytotoxicité :

Cette étude a été effectuée sur cellules L1210, voir Thèse Samadi-Baboli déjà citée.

L'addition de doses croissantes de LDL modifiées contenant de l'oléate d'elliptinium, a montré que les LDL modifiées sont nettement plus toxiques que l'oléate d'elliptinium ajouté seul à la même concentration.

Cette cytotoxicité dépend bien du récepteur pour les apolipoprotéines, puisqu'un excès de LDL natives ajouté à une concentration constante de LDL modifiées restaure la croissance cellulaire, alors qu'un excès de LDL méthylées, incapable de se fixer sur le récepteur, n'a aucun effet.

Les lipoprotéines selon l'invention peuvent être formulées avec tout excipient pharmaceutiquement ou cosmétiquement acceptable pour ainsi créer des formes galéniques ou pharmaceutiques classiques telles que des lotions, des solutions etc..

Les lipoprotéines peuvent être administrées par voie topique ou par voie parentérale et dans ce dernier cas peuvent être formulées dans un tampon injectable de préférence à raison de 0,05 à 1 %, et plus particulièrement de 0,1 à 0,4 %.

On peut utiliser par exemple un tampon phosphate ou carbonate éventuellement contenant un antioxidant tel que la vitamine E ou le probucol en une quantité suffisante pour prévenir l'oxidation.

### EXEMPLES DE FORMULATIONS

### a) Oléate d'elliptinium

| | |
|---|---|
| - LDL modifiées de l'exemple 1 | 0,5 % |
| - Tampon phosphate 10 mM, pH 7,4 avec vitamine E à 20 µM qsp | 100 % |

### b) Monolinolénate d'amétantrone

| | |
|---|---|
| - LDL modifiées de l'exemple 1 | 0,2 % |
| - Tampon carbonate 10 mM, pH 7,4 avec vitamine E à 20 µM qsp | 100 % |

### c) Dilinolénate d'amétantrone

| | |
|---|---|
| - LDL modifiées de l'exemple 1 | 0,2 % |
| - Tampon phosphate 10 mM, pH 7,4 avec vitamine E à 20 µM qsp | 100 % |

## Revendications

1. Procédé de préparation d'une lipoprotéine modifiée par incorporation d'au moins une substance active lipophile autre qu'un triglycéride et qu'un ester de cholestérol, caractérisé par le fait que l'on incorpore ladite substance active dans une émulsion d'une phase lipidique dans une phase continue aqueuse, que l'on ajoute à l'émulsion, en agitant, une lipoprotéine de départ et au moins une protéine de transfert de lipides, qu'on laisse incuber le mélange, et que l'on isole selon les méthodes connues la lipoprotéine dans laquelle la substance active est incorporée.

2. Procédé selon la revendication 1, caractérisé par le fait que la phase lipidique comprend au moins un triglycéride d'acide gras.

3. Procédé selon la revendication 1 ou 2, caractérisé par le fait que ladite phase lipidique comprend au moins un ester de cholestérol.

4. Procédé selon la revendication 1, caractérisé par le fait que ladite phase lipidique est constituée par des vésicules lipidiques telles que des liposomes ou analogues.

5. Procédé selon l'une quelconque des revendications précédentes, caractérisé par le fait que ladite émulsion est stabilisée à l'aide d'un agent émulsionnant.

6. Procédé selon la revendication 5, caractérisé par la fait que ledit agent émulsionnant est un phospholipide.

7. Procédé selon la revendication 6, caractérisé par la fait que ledit agent émulsionnant est la lécithine.

8. Procédé selon l'une quelconque des revendications précédentes, caractérisé par le fait que ladite lipoprotéine de départ est une lipoprotéine native.

9. Procédé selon la revendication 8, caractérisé par le fait que ladite lipoprotéine native est une LDL.

10. Procédé selon l'une quelconque des revendications 1 à 7, caractérisé par le fait que ladite lipoprotéine de départ est une lipoprotéine modifiée chimiquement.

11. Procédé selon la revendication 10, caractérisé par le fait que ladite lipoprotéine de départ est une LDL modifiée chimiquement pour permettre sa reconnaissance par le système réticulo-endothélial.

12. Procédé selon la revendication 11, caractérisé par le fait que ladite LDL de départ est une LDL acétylée, acétoacétylée, lactosylée ou oxydée.

13. Procédé selon l'une quelconque des revendications précédentes_{,} caractérisé par le fait que ladite lipoprotéine de départ est une lipoprotéine humaine.

14. Procédé selon l'une quelconque des revendications précédentes, caractérisé par la fait que ladite protéine de transfert de lipide est une protéine animale ou végétale.

15. Procédé selon la revendication 14, caractérisé par le fait que ladite protéine de transfert est ajoutée sous la forme d'une fraction plasmatique LPDS.

16. Procédé selon l'une quelconque des revendications précédentes, caractérisé par la fait que ladite substance active est une substance anti-tumorale, anti-proliférative, anti-parasitaire, anti-fongique, anti-bactérienne ou anti-virale, ou une vitamine liposoluble.

17. Procédé selon l'une quelconque des revendications précédentes caractérisé par le fait que la substance active lipophile est un médicament hydrophile modifié chimiquement pour être davantage lipophile.

## Claims

1. Method for preparing a lipoprotein modified by the incorporation of at least one lipophilic active substance other than a triglyceride and other than a cholesterol ester, characterised in that the said active substance is incorporated in an emulsion of a lipid phase in an aqueous continuous phase, in that a starting lipoprotein and at least one lipid-transfer protein are added to the emulsion with agitation, in that the mixture is allowed to incubate and in that the lipoprotein in which the active substance is incorporated is isolated according to known methods.

2. Method according to Claim 1, characterised in that the lipid phase comprises at least one fatty acid triglyceride.

3. Method according to Claim 1 or 2, characterised in that the said lipid phase comprises at least one cholesterol ester.

4. Method according to Claim 1, characterised in that the said lipid phase consists of lipid vesicles such as liposomes or the like.

5. Method according to any one of the preceding claims, characterised in that the said emulsion is stabilised using an emulsifying agent.

6. Method according to Claim 5, characterised in that the said emulsifying agent is a phospholipid.

7. Method according to Claim 6, characterised in that the said emulsifying agent is lecithin.

8. Method according to any one of the preceding claims, characterised in that the said starting lipoprotein in a native lipoprotein.

9. Method according to Claim 8, characterised in that the said native lipoprotein is an LDL.

10. Method according to any one of Claims 1 to 7, characterised in that the said starting lipoprotein is a chemically modified lipoprotein.

11. Method according to Claim 10, characterised in that the said starting lipoprotein is an LDL chemically modified in order to enable it to be recognised by the reticuloendothelial system.

12. Method according to Claim 11, characterised in that the said starting LDL is an acetylated, acetoacetylated, lactosylated or oxidised LDL.

13. Method according to any one of the preceding claims, characterised in that the said starting lipoprotein is a human lipoprotein.

14. Method according to any one of the preceding claims, characterised in that the said lipid-transfer protein is an animal or plant protein.

15. Method according to Claim 14, characterised in that the said transfer protein is added in the form of an LPDS plasma fraction.

16. Method according to any one of the preceding claims, characterised in that the said active substance is an antitumour, antiproliferative, antiparasitic, antifungal, antibacterial or antiviral substance or a fat-soluble vitamin.

17. Method according to any one of the preceding claims, characterised in that the lipophilic active substance is a hydrophilic medicinal product chemically modified in order to be more lipophilic.

## Patentansprüche

1. Verfahren zur Herstellung eines Lipoproteins, das durch das Inkorporieren mindestens eines lipophilen Wirkstoffes, der von einem Triglycerid und Cholesterinester verschieden ist, modifiziert wurde, dadurch gekennzeichnet, daß der Wirkstoff in eine Emulsion aus Lipidphase in kontinuierlichwäßriger Phase eingebracht wird, daß ferner zu der Emulsion unter Rühren ein Ausgangslipoprotein sowie mindestens ein Transferlipidprotein hinzugefügt wird, das Gemisch der Inkubierung unterzogen wird und anhand bekannter Verfahren das Lipoprotein, worin der Wirkstoff eingebaut worden ist, isoliert wird.

2. Verfahren nach Anspruch 1, dadurch gekennzeichnet, daß die Lipidphase mindestens ein Fettsäuretriglycerid aufweist.

3. Verfahren nach Anspruch 1 oder 2, dadurch gekennzeichnet, daß die Lipidphase mindestens einen Cholesterinester enthält.

4. Verfahren nach Anspruch 1, dadurch gekennzeichnet, daß die Lipidphase aus Lipidvesikeln wie z.B. Liposomen oder Analoga besteht.

5. Verfahren nach einem der vorstehenden Ansprüche, dadurch gekennzeichnet, daß die Emulsion mittels eines Emulgators stabilisiert wird.

6. Verfahren nach Anspruch 5, dadurch gekennzeichnet, daß der Emulgator ein Phosphorlipid ist.

7. Verfahren nach Anspruch 6, dadurch gekennzeichnet, daß der Emulgator in Form von Lecithin vorliegt.

8. Verfahren nach einem der vorstehenden Ansprüche, dadurch gekennzeichnet, daß das Ausgangslipoprotein ein natives Lipoprotein darstellt.

9. Verfahren nach Anspruch 8, dadurch gekennzeichnet, daß das native Lipoprotein ein LDL darstellt.

10. Verfahren nach einem der Ansprüche 1 bis 7, dadurch gekennzeichnet, daß das Ausgangslipoprotein ein chemisch modifiziertes Lipoprotein darstellt.

11. Verfahren nach Anspruch 10, dadurch gekennzeichnet, daß das Ausgangslipoprotein ein chemisch modifiziertes LDL darstellt, das zur Erkennung durch das retikuloendotheliale System befähigt ist.

12. Verfahren nach Anspruch 11, dadurch gekennzeichnet, daß das Ausgangs-LDL ein acetyliertes, acetoacetyliertes, lactosyliertes oder oxidiertes LDL darstellt.

13. Verfahren nach einem der vorstehenden Ansprüche, dadurch gekennzeichnet, daß das Ausgangslipoprotein ein Humanlipoprotein darstellt.

14. Verfahren nach einem der vorstehenden Ansprüche, dadurch gekennzeichnet, daß das Transferlipidprotein ein tierisches oder pflanzliches Protein darstellt.

15. Verfahren nach Anspruch 14, dadurch gekennzeichnet, daß das Transferprotein in Form einer Plasma-LPDS-(Lipoprotein Deficient Serum)Fraktion hinzugefügt wird.

16. Verfahren nach einem der vorstehenden Ansprüche, dadurch gekennzeichnet, daß der Wirkstoff eine antitumorale, antiproliferative, antiparasitäre, fungistatische, antibakterielle oder antiverale Substanz oder ein fettlösliches Vitamin darstellt.

17. Verfahren nach einem der vorstehenden Ansprüche, dadurch gekennzeichnet, daß der lipophile Wirkstoff einen chemisch modifizierten hydrophilen Arzneistoff darstellt, der verbesserte lipophile Eigenschaften aufweist.
